(19) Europäisches Patentamt
European Patent Office
Office européen des brevets

(11) **EP 2 908 720 B1**

(12) **EUROPÄISCHE PATENTSCHRIFT**

(45) Veröffentlichungstag und Bekanntmachung des Hinweises auf die Patenterteilung:
**06.11.2019 Patentblatt 2019/45**

(21) Anmeldenummer: **13774371.2**

(22) Anmeldetag: **20.09.2013**

(51) Int Cl.:
*A61B 5/024* (2006.01)  *A61B 5/0402* (2006.01)

(86) Internationale Anmeldenummer:
**PCT/EP2013/069647**

(87) Internationale Veröffentlichungsnummer:
**WO 2014/060182 (24.04.2014 Gazette 2014/17)**

(54) **VORRICHTUNG UND VERFAHREN ZUM ERKENNEN UND MELDEN EINES BELASTUNGSZUSTANDES EINER PERSON**

DEVICE AND METHOD FOR DETECTING AND SIGNALLING A STRESS STATE OF A PERSON

DISPOSITIF ET PROCÉDÉ POUR DÉTECTER ET SIGNALER UN ÉTAT DE TENSION D'UNE PERSONNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.10.2012 EP 12188701**

(43) Veröffentlichungstag der Anmeldung:
**26.08.2015 Patentblatt 2015/35**

(73) Patentinhaber: **Xotox Tools AG**
**8700 Küsnacht (CH)**

(72) Erfinder:
• BACHMANN, Philipp
  CH-8700 Küsnacht (CH)
• FRITZSCHE, Rolf
  CH-8340 Hinwil (CH)

(74) Vertreter: **Schmauder & Partner AG**
**Patent- & Markenanwälte VSP**
**Zwängiweg 7**
**8038 Zürich (CH)**

(56) Entgegenhaltungen:
**EP-A1- 2 316 333    DE-A1- 10 319 361**

**Beschreibung**

Technisches Gebiet

Die Erfindung betrifft eine Vorrichtung und ein Verfahren zum Erkennen und Melden eines Belastungszustandes einer Person.

Stand der Technik

Für die Ermittlung von Stresszuständen die Pulsrate oder die Herzratenvariabilität zu verwenden, ist seid langem bekannt. Dabei wird der Abstand zwischen zwei Herzschlägen - im Sinne dieser Erfindung - definiert als die Zeit zwischen dem Beginn zweier Kontraktionen der Herzkammern. Dieser Beginn der Kammerkontraktion wird im Elektrokardiogramm (EKG) als R-Zacke dargestellt. Der Abstand zwischen zwei R-Zacken wird üblicherweise als RR-Intervall bezeichnet. Nach einer Mittelung über eine bestimmte Anzahl von RR-Intervallen kann man rechnerisch die Herzfrequenz bestimmen. Die einzelnen Werte der RR-Intervalle schwanken um den so erhaltenen Mittelwert. Dabei können sich die Abweichungen von Schlag zu Schlag ändern. Die Abweichung wird zumeist als Herzratenvariabilität (HRV) bezeichnet. Grundsätzlich kann die Herzfrequenz auch durch eine Druckmessung an einer Arterie vorgenommen werden.

Physiologisch hängt die Herzratenvariabilität (HRV) mit der Fähigkeit des menschlichen Organismus zusammen, die Frequenz des Herzrhythmus anzupassen. Herzratenänderungen, also Veränderungen des zeitlichen Abstandes zwischen zwei Herzschlägen, treten sowohl im Ruhezustand, dann zumeist spontan, wie auch bei bestimmten Veränderungen des Umfeldes, z.B. Belastungen auf. Über physiologische Regulationswege des vegetativen Nervensystems passt ein gesunder Organismus die Herzschlagrate beständig momentanen Erfordernissen an. Körperliche Beanspruchung oder psychische Belastung hat deswegen in der Regel eine Erhöhung der Herzfrequenz zur Folge, die bei Entlastung und Entspannung normalerweise wieder zurückgeht. Dabei zeigt sich eine gute Anpassungsfähigkeit an Belastungen in einer höheren Variabilität der Herzfrequenz. Unter chronischer Stressbelastung ist die Anpassungsfähigkeit reduziert. Insofern bietet - dies ist bekannt - die Herzratenvariabilität für sich genommen schon einen gewissen, allerdings noch sehr unzuverlässigen Indikator für die momentane Stressbelastungen sowie die Stressbelastungsfähigkeit einer Person.

Im Stand der Technik wurden vielfach Verfahren zur Bestimmung des Belastungszustandes (Stresszustandes) einer Person vorgeschlagen, wobei vorgeschlagen wurde, neben der Pulsrate weitere Messgrössen zu verwenden. So wird in der DE 103 19 361 A1 vorgeschlagen, neben der Herzratenvariabilität die Pulswellenlatenz zu verwenden.

In Bezug auf die Auswertung der Herzratenvariabilität wird auf die DE 100 06 154 A1, die DE 10 2006 039 957 A1, sowie die DE 10 2008 030 956 A1 und die EP 1 156 851 B1 verwiesen, in denen der Fachmann verschiedene Verfahren zur Bestimmung finden kann.

Aus der EP-2 316 333 A1 ist eine Vorrichtung und ein Verfahren bekannt, bei der eine Zustandsgrösse berechnet wird, die eine Funktion der momentanen Pulsfrequenz und der momentanen Herzratenvariabilität ist, vorzugsweise eine Linearkombination der momentanen Pulsfrequenz P und der momentanen Herzratenvariabilität ist. Weiterhin ist in der genannten Druckschrift vorgeschlagen worden, die Zustandsfunktion mit zumindest einer Korrekturgrösse zu versehen, die die Vorgeschichte der Person zumindest innerhalb der letzten 0,5 Stunden berücksichtigt.

Dieser Ansatz erscheint dabei durchaus geeignet. Es ergibt sich aber ein weiteres Optimierungsproblem, welche dadurch beschrieben werden kann, dass einerseits - insbesondere zum Vergleich verschiedener Personen - Normierungen eingeführt werden müssen, aber andererseits auch individuelle Unterschiede berücksichtigt werden sollen.

Darstellung der Erfindung

Aufgabe der Erfindung ist es, eine Vorrichtung und ein entsprechendes Verfahren zum Erkennen und Melden eines Belastungszustandes einer Person mit einer - gegenüber dem Stand der Technik - erhöhten Zuverlässigkeit zur Verfügung zu stellen und bei denen einerseits - insbesondere zum Vergleich verschiedener Personen - Normierungen eingeführt werden können, aber andererseits auch individuelle Unterschiede berücksichtigt werden.

Die Aufgabe der Erfindung wird - gemäss einem ersten Aspekt - durch ein Verfahren nach Anspruch 1 gelöst. Die Massnahmen der Erfindung haben zunächst einmal zur Folge, dass in einer ersten Näherung, nämlich im ersten Verarbeitungsfenster, also in einem ersten Zeitintervall $T_1$ oder einer vorgegebenen Anzahl von Pulsschlägen, ein erstes Näherungsergebnis erzielt wird, mit welchem bereits gearbeitet werden kann. Hierzu bieten sich insbesondere die Alterswerte an, die - vorzugsweise in jeweiligen Altersgruppen - typische Tabellenwerte für die Minimal- und die Maximalwerte der Pulsrate und des HRV-Wertes liefern. Die weiteren Verarbeitungsfenster können dann - gemäss experimentellen Studien - Ergebnisse liefern, die insbesondere dann verbessert sind, wenn die Person in ihrer Charakteristik von den Tabellenwerten für die Minimal- und die Maximalwerte der Pulsrate und des HRV-Wertes abweichen, sei es aus Gründen z.B. des besonders guten oder besonders schlechten Trainingsstandes oder auch wegen hoher oder niedriger Stresswerte in der näheren oder mittleren Historie. Es sollte bemerkt werden, dass die Pulsaufnahme irgendwo am Körper möglich ist, wobei man grundsätzlich optisch, akustisch oder kinästhetisch messen und in elektrische Signale umwandeln kann. Auch eine direkte Messung elektrischer Signale zur Pulsmessung ist für die Erfindung möglich.

Entsprechend dem Verfahren zum Erkennen und Melden eines Belastungszustandes einer Person gemäss der vorliegenden Erfindung werden dabei folgende Schritte durchgeführt:
Es werden in dem Zeitintervall oder für eine vorgegebene Zahl von Pulsschlägen fortlaufend die Daten der momentanen Pulsfrequenz P und der momentanen Herzratenvariabilität HRV erfasst und verarbeitet. Dabei wird getrennt für die Pulsrate und die Herzratenvariabilität ein Wert für den Stressindex berechnet und anschliessend die beiden Werte - mit Gewichtungsfaktoren - addiert. Die Werte der Pulsraten und der Herzratenvariabilität werden dabei mit Hilfe von - vorzugsweise altermässig sortierten - tabellenmässig erwarteten Extremwerten $P_{max}$, $P_{min}$, $HRV_{max}$ und $HRV_{min}$ normiert. Damit wird ein erster- möglicherweise schon brauchbarer Wert - für den Stressindex ermittelt.

Weiter wird aber für das Zeitintervall oder die vorbestimmte Zahl von Pulsschlägen ermittelt, ob die persönlichen Extremwerte $P_{max}$ ,$P_{min}$, $HRV_{max}$ und $HRV_{min}$ anders sind als die zuvor verwendeten Werte. Für das nächste Zeitintervall oder für die nächste Messung von vorgegebenen Pulsschlägen, wobei weder die Länge des Zeitintervalls noch die Zahl von Pulsschlägen notwendigerweise identisch sein müssen wie für die erste Messung, und welches auch deutlich später, also mit einem zeitlichen Zwischenraum von bis zu mehreren Stunden liegen kann, werden dann entweder die alten Werte oder die neu ermittelten Extremwerte verwendet, je nachdem, welche Werte extremer sind. Die Berechnung des neuen Wertes für den Stressindex geschieht dann prinzipiell ebenso wie die beschriebene erste Messung, also fortlaufendes getrenntes Ermitteln der Stressindexwerte für die Pulsrate und die Herzratenvariabilität ein Wert für den Stressindex berechnet und anschliessendes addieren der beiden Werte mit Gewichtungsfaktoren. Das Zeitintervall wird dabei typischerweise im Bereich von 100s bis 1000 s - vorzugsweise 300s bis 500s - liegen, kann aber auch jeweils um eine Grössenordnung (Faktor 10) kleiner oder grösser sein. Die Zahl der Pulsschläge wird zwischen 50 und 500, vorzugsweise 100 liegen, kann aber insbesondere auch um ein bis zwei Grössenordnungen grösser sein. Es sollte betont werden, dass die Folgemessungen sich durchaus auch zeitlich überlappen können, wobei dann bei Beginn der neuen Messreihe selbstverständlich nur die Extremwerte verwendet werden können, die zuvor aufgetreten sind. Diese "Movingwindow" Methode kann dann sinnvoll sein, wenn sehr viel Rechenkapazität zur Verfügung steht und ein sehr schnelles Ergebnis erzielt werden soll.

Vorteilhaft ist das Verfahren durchführbar, wenn die Normierung jeweils mittels eines Normierungswertes bei der ersten, tabellenbezogenen

$$P_z = \quad P_{min} + a*(P_{max} - P_{min})$$

$$HRV_z = HRV_{min} + b* (HRV_{max} - HRV_{min})$$

und die Berechnung der Summanden für des Stresswert SI jeweils mittels

$$SI_P = (P_{d1} - P_z)/(P_{max} - P_z) \text{ wenn } P_{d1} > P_z$$

$$SI_P = (P_{d1} - P_z)/(P_z - P_{min}) \text{ wenn } P_{d1} < P_z$$

$$SI_{HRV} = -(HRV_{d1} - HRV_z)/(HRV_{max} - HRV_z) \text{ wenn } HRV_{d1} > HRV_z$$

$$SI_{HRV} = -(HRV_{d1} - HRV_z)/(HRV_z - HRV_{min}) \text{ wenn } HRV_{d1} < HRV_z$$

erfolgt.

Die weiteren Fenster werden dann vorteilhafterweise berechnet gemäss

$$SI_P = (P_{dx} - P_z)/(P_{max} - P_z) \text{ wenn } P_{dx} > P_z$$

$$SI_P = (P_{dx} - P_z)/(P_z - P_{min}) \text{ wenn } P_{dx} < P_z$$

$$SI_{HRV} = -(HRV_{dx} - HRV_z)/(HRV_{max} - HRV_z) \text{ wenn } HRV_{dx} > HRV_z$$

$$SI_{HRV} = -(HRV_{dx} - HRV_z)/(HRV_z - HRV_{min}) \text{ wenn } HRV_{dx} < HRV_z.$$

Vorteilhaft hat sich herausgestellt, wenn die Werte a für die Verarbeitung der Pulsrate gewählt wird im Bereich zwischen 0.2 und 0.3, vorteilhafterweise als 0.25 und die Werte b für die Verarbeitung der Herzratenvariabilität zwischen 0.33 bis 0.66, vorteilhafterweise als 0.5.

Das Verfahren ist - je nach Anwendungsfall - durchaus kalibrierbar in dem Sinne, dass die Gewichtung des Stressindexteilwertes, der aus der Pulsrate ermittelt wird und dem Stressindexteilwert, der aus der Herzratenvariabilität ermittelt wird, durch Tests optimiert wird. Sofern dazu keine Veranlassung besteht, wird man die Werte c und d wählen als 1.

Besonders vorteilhaft ist es, wenn die jeweiligen ermittelten Stresswerte nicht nur direkt ausgegeben werden, was aber selbstverständlich nicht ausgeschlossen ist, sondern der aktuelle Stressindex SI nach dem Zeitintervall $T_x$ oder nach der vorgegebenen Anzahl von Pulsschlägen einem Filter, typischerweise einem digitalen Tiefpass, zugeführt wird gewählt wird, um einzelne Ausreisser mitteln zu können, z.B. SI = $f^*SI_x + (1-f)^*SI_{x-1}$, mit f zwischen 0.05 und 0.5, vorteilhafterweise von 0.1.

Gemäss einem zweiten Aspekt der vorliegenden Erfindung wird eine Vorrichtung vorgeschlagen, die zur Durchführung des erfindungsgemässen Verfahrens, einschliesslich der vorstehend genannten vorteilshaften Ausführungsformen, möglicherweise aber auch ohne diese, geeignet ist.

Eine solche Vorrichtung zum Erkennen und Melden eines Belastungszustandes einer Person, wird typischer Weise eine Erfassungseinrichtung zum fortlaufenden Erfassen der Daten der momentanen Pulsfrequenz und der momentanen Herzratenvariabilität, eine Verarbeitungseinrichtung zum fortlaufenden Verarbeiten der Daten der momentanen Pulsfrequenz und der momentanen Herzratenvariabilität, und eine Vergleichseinrichtung zum Vergleichen der gewonnenen momentanen Zustandsfunktion der Person mit einem Alarmkriterium umfassen.

Die vorbenannten sowie die beanspruchten und in den nachfolgenden Ausführungsbeispielen beschriebenen, erfindungsgemäss zu verwendenden Elemente unterliegen in ihrer Grösse, Formgestaltung, Materialverwendung und ihrer technischen Konzeption keinen besonderen Ausnahmebedingungen, so dass die in dem jeweiligen Anwendungsgebiet bekannten Auswahlkriterien uneingeschränkt Anwendung finden können. Die Berechnung der Herzratenvariabilität kann zur Durchführung des erfindungsgemässen Verfahrens durch übliche Methoden (z.B. in der Zeitdomäne: RMSSD, RRinter, SDNN, oder in der Frequenzdomäne $LF_{tot}/HF_{tot}$) durchgeführt werden, wobei die Methode RMSSD ("Root mean square of sucsessive differences") für die hier genannten Methode und Vorrichtung geeignet ist. Es solle darauf hingewiesen werden, dass das erfinderische Verfahren nicht dazu gedacht, den Gesundheits- oder Krankheitszustand der Person festzustellen, während die erfindungsgemässe Vorrichtung diesbezüglich keiner Einschränkung unterliegt.

Wege zur Ausführung der Erfindung

Die erfindungsgemässe Vorrichtung umfasst gemäss einem bevorzugten Ausführungsbeispiel der Erfindung eine Messeinrichtung zur Ermittlung der Pulsrate und der Werte, die zur Berechnung einer Herzratenvariabilität notwendig sind. Dies ist hier ein Pulsmesssensor, kann aber auch alternativ ein elektrischer Sensor zur Messung von elektrischen kardiografischen Messwerten sein, sowie ein Anzeigegerät. Weiterhin umfasst die Vorrichtung eine Schnittstelle zur Eingabe von personenbezogenen Grössen, die insbesondere zur Ermittlung der erfindungsgemäss zu verwendenden Historie notwendig sind. Kernstück der Vorrichtung ist eine Recheneinrichtung, die die notwendige Messdatenerfassung kontrolliert, die Messdatenaufbereitung in der notwendigen digitalen Form durchführt, die Datenverarbeitung durchführt und die Anzeige bedient.

Im vorliegenden Ausführungsbeispiel wird die Herzratenvariablilität HRV bestimmt durch die RMSSD-Methode ("Root mean square of sucsessive differences"), wobei aber auch andere Methoden, wie z.B. die Methode "SDRR" mit Hilfe von Standardabweichungen, die Methode pRR50 bei der die Anzahl der aufeinanderfolgenden RR-Intervalle ermittelt wird, die grösser als 50ms, und dieser Wert dividiert durch die Gesamtzahl aufeinanderfolgender RR-Intervalle oder Frequenzorientierte Methoden wie beispielsweise die Berechnung über die Quotienten $LF_{tot}/HF_{tot}$ der niederfrequentigen Frequenzanteile durch die höherfrequentigen Frequenzanteile. Der HRV Wert mittels RMSSD wird berechnet als die Quadratwurzel der Summe der quadrierten Differenzen zwischen benachbarten RR-Intervallen. In diesem Zusammenhang sollte darauf hingewiesen werden, dass bei der Wahl der Berechnungsmethode einerseits die jeweiligen neueren Erkenntnisse des jeweiligen Fachgebietes bzw. des jeweiligen Einsatzspektrums des erfindungsgemässen Verfahren bzw. der erfindungsgemässen Vorrichtung berücksichtigt werden können, aber andererseits auch rein praktische Gesichtspunkte der jeweiligen Auswahl denkbar sind. Im Falle der Ermittlung der HRV-Werte durch die RMSSD-Methode wird im hier vorliegenden Ausführungsbeispiel als Tabellenwert für $HRV_{min}$ für jedes Alter als 0 eingesetzt. Die im hier gezeigten Ausführungsbeispiel verwendeten anderen Minimalwerte für den Puls bzw. HRV werden gemäss der folgenden Tabelle gewählt:

Altersabhängige Ruhepulswerte

| | |
|---|---|
| Jugendlich: 14...18 | Ruhepuls: 85 Schläge/Minut |
| Erwachsene: 19...65 | Ruhepuls: 70 Schläge/Minute |
| Senioren: 65+ | Ruhepuls: 90 Schläge/Minute |

Altersabhängige $HRV_{max}$ Werte
(RMSSD)

| | |
|---|---|
| 15...20 | 47ms |
| 21...30 | 46ms |
| 31...40 | 40ms |
| 41...50 | 35ms |
| 51...60 | 30ms |
| 61...70 | 24ms |

nach Angelink et al: Innovationstagung FH Rapperswil 4.5.2011

Es sollte dabei beachtet werden, dass - ohne den Sinn des erfindungsgemässen Verfahren zu verlassen, durchaus auch andere Parameter zu den Probanden, wie z.B. das Geschlecht etc. in die Tabelle einfliessen können.

Gemäss dem Ausführungsbeispiel wird die Normierung jeweils mittels eines Normierungswertes

$$P_z = P_{min} + a*(P_{max} - P_{min})$$

$$HRV_z = HRV_{min} + b*(HRV_{max} - HRV_{min})$$

und die Berechnung der Summanden für des Stresswert SI jeweils mittels

$$SI_P = (P_{d1} - P_z)/(P_{max} - P_z) \text{ wenn } P_{d1} > P_z$$

$$SI_P = (P_{d1} - P_z)/(P_z - P_{min}) \text{ wenn } P_{d1} < P_z$$

$$SI_{HRV} = -(HRV_{d1} - HRV_z)/(HRV_{max} - HRV_z) \text{ wenn } HRV_{d1} > HRV_z$$

$$SI_{HRV} = -(HRV_{d1} - HRV_z)/(HRV_z - HRV_{min}) \text{ wenn } HRV_{d1} < HRV_z$$

bzw.

$$SI_P = (P_{dx} - P_z)/(P_{max} - P_z) \text{ wenn } P_{dx} > P_z$$

$$SI_P=(P_{dx}-P_z)/(P_z-P_{min}) \text{ wenn } P_{dx} < P_z$$

$$SI_{HRV}=-(HRV_{dx}-HRV_z)/(HRV_{max}-HRV_z) \text{ wenn } HRV_{dx} > HRV_z$$

$$SI_{HRV}=-(HRV_{dx}-HRV_z)/(HRV_z-HRV_{min}) \text{ wenn } HRV_{dx} < HRV_z$$

durchgeführt, wobei a gewählt wurde als 0.25 und b als 0.5 und c und d gewählt als 1. Die aktuellen Werte des Stressindex SI nach der vorgegebenen Anzahl von Pulsschlägen gewählt werden im Ausführungsbeispiel mittels eines digitalen Tiefpassfilter $SI = f*SI_x+(1-f)*SI_{x-1}$ berechnet mit f von 0.1. Die Vorrichtung wurde entsprechend eingerichtet.

Es sollte noch bemerkt werden, dass die einzelnen Fenster, in denen die Stressindexwerte ermittelt werden, vorteilhafterweise für diverse Zustände des Probanden einschliessen, wie z.B. liegend, stehend, sich - im Sinne des Conconitest - bewegend etc.

Weiterhin sollte darauf hingewiesen werden, dass selbst bei zeitlich sehr weit auseinanderliegenden Testintervallen die Übernahme des letzten Testintervalls zu einem besseren bzw. zu einem schneller guten Ergebnis führt als wenn man mit den Tabellenwerten starten muss. Andererseits kann es selbstverständlich angezeigt sein, wieder Tabellenwerte zu benutzen, wenn sich der Zustand des Probanden grundsätzlich erheblich geändert hat.

## Patentansprüche

1. Verfahren zum Erkennen und Melden eines Belastungszustandes einer Person, wobei das Verfahren folgende Schritte umfasst:

   • fortlaufendes Erfassen der Daten der momentanen Pulsfrequenz P und der momentanen Herzratenvariabilität HRV, mittels eines Pulsmesssensors oder eines elektrokardiografischen Sensors;
   • fortlaufendes Verarbeiten der Daten der momentanen Pulsfrequenz P und der momentanen Herzratenvariabilität HRV,
   • Bestimmen eines Stressindexes und Vergleichen desselben mit einem Alarmkriterium,

   **dadurch gekennzeichnet, dass das Bestimmen des Belastungszustandes umfasst:**

   in einem ersten Zeitintervall $T_1$ oder einer vorgegebenen Anzahl von Pulsschlägen ein erster Wert $SI_1$ für den Stressindex bestimmt wird durch die Addition eines Wertes $SI_P$ für den Stressindex gewonnen aus einem normierten Durchschnittswert $P_{d1}$ des Pulses im genannten ersten Zeitintervall $T_1$ oder über die vorgegebene Anzahl von Pulsschlägen und eines Wertes $SI_{HRV}$ gewonnen aus einem normierten Durchschnittswert $HRV_{d1}$ der Herzratenvariabilität HRV im genannten ersten Zeitintervall $T_1$ oder über die vorgegebene Anzahl von Pulsschlägen gemäss

   $$SI_1= c* SI_P + d* SI_{HRV}$$

   wobei c und d Gewichtungsfaktoren des Stressindexteilwertes sind, der aus der Pulsrate bzw. dem Stressindexteilwert, der aus der Herzratenvariabilität ermittelt wird, wobei die Normierung mittels Tabellenwerten $P_{max}$, $P_{min}$, $HRV_{max}$ und $HRV_{min}$ durchgeführt wird mittels aus altersabhängigen Minimal- und Maximal Puls und HRV-Werten, sowie die Maximal- und Minimalwerte der gemessenen Pulswerte und der HRV-Werte im Zeitintervall $T_1$ oder während der vorgegebenen Anzahl von Pulsschlägen bestimmt werden,

   wobei $T_1$ zwischen 100s und 1000 s, vorzugsweise mit 300s, oder die vorgegebene Anzahl von Pulsschlägen zwischen 50 und 500 liegt und vorzugsweise 100 ist,

   in zumindest einem weiteren Zeitintervall $T_x$(x= 2...n) oder während einer weiteren vorgegebenen Anzahl von Pulsschlägen ein weiterer Wert $SI_x$ für den Stressindex bestimmt wird durch die Addition eines Wertes $SI_P$ für den Stressindex gewonnen aus einem normierten Durchschnittswert $P_{d1}$ des Pulses im genannten Zeitintervall $T_x$ oder über die vorgegebene Anzahl von Pulsschlägen und eines Wertes $SI_{HRV}$ gewonnen aus einem normierten Durchschnittswert $HRV_{d1}$ der Herzratenvariabilität HRV im genannten Zeitintervall $T_x$ oder über die vorgegebene Anzahl von Pulsschlägen

$$SI_x = c^* \ SI_P + d^* \ SI_{HRV}$$

vorzugsweise mit gleicher Länge wie $T_1$ oder bei gleicher Anzahl von Pulsschlägen, wobei c und d wiederum die Gewichtungsfaktoren des Stressindexteilwertes sind, der aus der Pulsrate bzw. dem Stressindexteilwert, der aus der Herzratenvariabilität ermittelt wird,
wobei die Normierung mittels Werten $P_{max}$, $P_{min}$, $HRV_{max}$ und $HRV_{min}$ durchgeführt wird, wobei $P_{max}$ bzw. $HRV_{max}$ gewählt werden aus dem grösseren Wert von $P_{max}$ bzw $HRV_{max}$ bestimmt im vorherigen Zeitintervall $T_{x-1}$, oder während der vorherigen, vorgegebenen Anzahl von Pulsschlägen und dem vorherigen Zeitintervall $T_{x-1}$ oder während der vorgegebenen Anzahl von Pulsschlägen verwendeten Wert von $P_{max}$ bzw $HRV_{max}$, $P_{min}$ bzw. $HRV_{min}$ gewählt werden aus dem kleineren Wert von $P_{min}$ bzw.
$HRV_{min}$ aus dem vorherigen Zeitintervall $T_{x-1}$ oder während der vorherigen, vorgegebenen Anzahl von Pulsschlägen und dem vorherigen Zeitintervall $T_{x-1}$ oder während der vorherigen, vorgegebenen Anzahl von Pulsschlägen verwendeten Wert von $P_{min}$ bzw. $HRV_{min}$, wobei der so gewonnene weitere Wert $SI_x$ für den Stressindex dem zu bestimmenden Stressindex entspricht.

2. Verfahren zum Erkennen und Melden eines Belastungszustandes einer Person nach Anspruch 1, wobei die Normierung jeweils mittels eines Normierungswertes

$$P_z = \quad P_{min} + a^*(P_{max} - P_{min})$$

$$HRV_z = HRV_{min} + b^* \ (HRV_{max} - HRV_{min})$$

und die Berechnung der Summanden für des Stresswert SI jeweils mittels

$$SI_P = (P_{d1} - P_z)/(P_{max} - P_z) \text{ wenn } P_{d1} > P_z$$

$$SI_P = (P_{d1} - P_z)/(P_z - P_{min}) \text{ wenn } P_{d1} < P_z$$

$$SI_{HRV} = -(HRV_{d1} - HRV_z)/(HRV_{max} - HRV_z) \text{ wenn } HRV_{d1} > HRV_z$$

$$SI_{HRV} = -(HRV_{d1} - HRV_z)/(HRV_z - HRV_{min}) \text{ wenn } HRV_{d1} < HRV_z$$

bzw.

$$SI_P = (P_{dx} - P_z)/(P_{max} - P_z) \text{ wenn } P_{dx} > P_z$$

$$SI_P = (P_{dx} - P_z)/(P_z - P_{min}) \text{ wenn } P_{dx} < P_z$$

$$SI_{HRV} = -(HRV_{dx} - HRV_z)/(HRV_{max} - HRV_z) \text{ wenn } HRV_{dx} > HRV_z$$

$$SI_{HRV} = -(HRV_{dx} - HRV_z)/(HRV_z - HRV_{min}) \text{ wenn } HRV_{dx} < HRV_z$$

durchgeführt wird.

3. Verfahren nach Anspruch 2, wobei a gewählt wird als 0.25 und b als 0.5.

4. Verfahren nach einem der Ansprüche 1 bis 3, wobei c und d gewählt werden als 1.

**5.** Verfahren nach einem der Ansprüche 1 bis 4, wobei der aktuelle Stressindex SI nach dem Zeitintervall $T_x$ oder nach der vorgegebenen Anzahl von Pulsschlägen gewählt wird mittels eines digitalen Tiefpassfilter $SI = f*SI_x + (1-f)*SI_{x-1}$ mit f zwischen 0.05 und 0.5, vorzugsweise von 0.1.

**6.** Verfahren nach einem der Ansprüche 1 bis 5, wobei sich die Zeitintervalle bzw. die Zeiten, in denen eine vorgegebene Anzahl von Pulsschlägen gemessen wird, überlappen.

**7.** Verfahren nach einem der Ansprüche 1 bis 5, wobei die Zeitintervalle bzw. die Zeiten, in denen eine vorgegebene Anzahl von Pulsschlägen gemessen wird, untereinander einen festen oder variablen Abstand haben.

**8.** Vorrichtung zum Erkennen und Melden eines Belastungszustandes einer Person, mit:

• einer Erfassungseinrichtung zum fortlaufenden Erfassen der Daten der momentanen Pulsfrequenz und der momentanen Herzratenvariabilität, wobei die besagte Erfassungseinrichtung ein Pulsmesssensor oder ein elektrokardiografischer Sensor ist,
• einer Verarbeitungseinrichtung zum fortlaufenden Verarbeiten der Daten der momentanen Pulsfrequenz und der momentanen Herzratenvariabilität, und
• einer Vergleichseinrichtung zum Bestimmen eines Stressindexes und zum Vergleichen desselben mit einem Alarmkriterium,

**dadurch gekennzeichnet, dass**
die Verarbeitungseinrichtung so eingerichtet ist, dass in einem ersten Zeitintervall $T_1$ oder einer vorgegebenen Anzahl von Pulsschlägen ein erster Wert $SI_1$ für den Stressindex bestimmt wird durch die Addition eines Wertes $SI_P$ für den Stressindex gewonnen aus einem normierten Durchschnittswert $P_{d1}$ des Pulses im genannten ersten Zeitintervall $T_1$ oder über die vorgegebene Anzahl von Pulsschlägen und eines Wertes $SI_{HRV}$ gewonnen aus einem normierten Durchschnittswert $HRV_{d1}$ der Herzratenvariabilität HRV im genannten ersten Zeitintervall $T_1$ oder über die vorgegebene Anzahl von Pulsschlägen

$$SI_1 = c* SI_P + d* SI_{HRV}$$

wobei c und d Gewichtungsfaktoren des Stressindexteilwertes sind, der aus der Pulsrate bzw. dem Stressindexteilwert, der aus der Herzratenvariabilität ermittelt wird, wobei die Normierung mittels Tabellenwerten $P_{max}$, $P_{min}$, $HRV_{max}$ und $HRV_{min}$ durchgeführt wird mittels aus altersabhängigen Minimal- und Maximal Puls und HRV-Werten, sowie die Maximal- und Minimalwerte der gemessenen Pulswerte und der HRV-Werte im Zeitintervall $T_1$ oder während der vorgegebenen Anzahl von Pulsschlägen bestimmt werden,
wobei $T_1$ zwischen 100s und 1000 s, vorzugsweise mit 300s, oder die vorgegebene Anzahl von Pulsschlägen zwischen 50 und 500 liegt und vorzugsweise 100 ist,
in zumindest einem weiteren Zeitintervall $T_x(x= 2...n)$ oder während einer weiteren vorgegebenen Anzahl von Pulsschlägen ein weiterer Wert $SI_x$ für den Stressindex bestimmt wird durch die Addition eines Wertes $SI_P$ für den Stressindex gewonnen aus einem normierten Durchschnittswert $P_{d1}$ des Pulses im genannten Zeitintervall $T_x$ oder über die vorgegebene Anzahl von Pulsschlägen und eines Wertes $SI_{HRV}$ gewonnen aus einem normierten Durchschnittswert $HRV_{d1}$ der Herzratenvariabilität HRV im genannten Zeitintervall $T_x$ oder über die vorgegebene Anzahl von Pulsschlägen

$$SI_x = c* SI_P + d* SI_{HRV}$$

vorzugsweise mit gleicher Länge wie $T_1$ oder bei gleicher Anzahl von Pulsschlägen, wobei c und d wiederum die Gewichtungsfaktoren des Stressindexteilwertes sind, der aus der Pulsrate bzw. dem Stressindexteilwert, der aus der Herzratenvariabilität ermittelt wird,
wobei die Normierung mittels Werten $P_{max}$, $P_{min}$, $HRV_{max}$ und $HRV_{min}$ durchgeführt wird, wobei $P_{max}$ bzw. $HRV_{max}$ gewählt werden aus dem grösseren Wert von $P_{max}$ bzw $HRV_{max}$ bestimmt im vorherigen Zeitintervall $T_{x-1}$, oder während der vorherigen, vorgegebenen Anzahl von Pulsschlägen und dem vorherigen Zeitintervall $T_{x-1}$ oder während der vorgegebenen Anzahl von Pulsschlägen verwendeten Wert von $P_{max}$ bzw $HRV_{max}$, $P_{min}$ bzw. $HRV_{min}$ gewählt werden aus dem kleineren Wert von $P_{min}$ bzw. $HRV_{min}$ aus dem vorherigen Zeitintervall $T_{x-1}$ oder während der vorherigen, vorgegebenen Anzahl von Pulsschlägen und dem vorherigen Zeitintervall $T_{x-1}$ oder während der vorherigen, vorgegebenen Anzahl von Pulsschlägen verwendeten Wert von $P_{min}$ bzw. $HRV_{min}$, wobei der so gewonnene

weitere Wert $SI_x$ für den Stressindex dem zu bestimmenden Stressindex entspricht.

9. Vorrichtung nach Anspruch 8, wobei die Vorrichtung so eingerichtet ist, dass das Verfahren gemäss Anspruch 2 durchgeführt werden kann.

10. Vorrichtung nach Anspruch 8 oder 9, wobei a gewählt wird als 0.25 und b als 0.5 und /oder wobei c und d gewählt werden als 1 und/oder der aktuelle Stressindex SI nach dem Zeitintervall $T_x$ oder nach der vorgegebenen Anzahl von Pulsschlägen gewählt wird mittels eines digitalen Tiefpassfilter $SI = f*SI_x+(1-f)*SI_{x-1}$
mit f zwischen 0.05 und 0.5, vorzugsweise von 0.1.

**Claims**

1. A method for detecting and reporting of a stress condition of a person, wherein the method comprises the following steps:

   • continuously acquiring data of the current pulse frequency P and of the current heart rate variability HRV by means of a pulse sensor or an electrocardiographic sensor,
   • continuously processing the data of the current pulse frequency P and of the current heart rate variability HRV,
   • determining a stress index and comparing the same with an alarm criterion,

   **characterized in that determining of the stress condition comprises:**

   within a first time interval $T_1$ or across a predetermined number of pulse beats a first value $SI_1$ for the stress index is determined by adding a value $SI_P$ for the stress index, which is obtained from a normalized average value $P_{d1}$ of the pulse frequency in the mentioned first time interval $T_1$ or across the predetermined number of pulse beats, plus a value $SI_{HRV}$, which is obtained from a normalized average value $HRV_{d1}$ of the heart rate variability HRV within the mentioned first time interval $T_1$ or across the predetermined number of pulse beats according to:

   $$SI_1 = c* SI_P + d* SI_{HRV}$$

   wherein c and d are weighting factors of the stress index partial value which is determined from the pulse frequency and of the stress index partial value which is determined from the heart rate variability, respectively, wherein normalization is carried out by means of tabulated values $P_{max}$, $P_{min}$, $HRV_{max}$ and $HRV_{min}$ obtained from age dependent minimum and maximum pulse frequency values and HRV values, and, furthermore, the maximum and minimum values of the measured pulse frequency values and HRV values within the time interval $T_1$ or across the predetermined number of pulse beats are determined,
   wherein $T_1$ lies between 100 s and 1000 s, and is preferably 300 s, or the predetermined number of pulse beats lies between 50 and 500, and is preferably 100,
   in at least one further time interval $T_x (x= 2...n)$ or across a further predetermined number of pulse beats a further value $SI_x$ for the stress index is determined by adding a value $SI_P$ for the stress index, which is obtained from a normalized average value $P_{d1}$ of the pulse frequency in the mentioned time interval $T_x$ or across the predetermined number of pulse beats, plus a value $SI_{HRV}$, which is obtained from a normalized average value $HRV_{d1}$ of the heart rate variability HRV within the mentioned time interval $T_x$ or across the predetermined number of pulse beats:

   $$SI_x = c* SI_P + d* SI_{HRV}$$

   preferably having the same length as $T_1$ or having the same number of pulse beats, wherein c and d are again weighting factors of the stress index partial value which is determined from the pulse frequency and of the stress index partial value which is determined from the heart rate variability, respectively,
   wherein normalization is carried out by means of values $P_{max}$, $P_{min}$, $HRV_{max}$ and $HRV_{min}$, wherein $P_{max}$ and $HRV_{max}$ are selected from the larger value of $P_{max}$ and $HRV_{max}$ determined in the previous time interval $T_{x-1}$ or across the predetermined number of pulse beats and the values of $P_{max}$ and $HRV_{max}$ used in the previous time interval $T_{x-1}$ or across the predetermined number of pulse beats, and wherein $P_{min}$ and $HRV_{min}$ are selected

from the smaller value of $P_{min}$ and $HRV_{min}$ determined in the previous time interval $T_{x-1}$ or across the predetermined number of pulse beats and the values of $P_{min}$ and $HRV_{min}$ used in the previous time interval $T_{x-1}$ or across the predetermined number of pulse beats, wherein the further value $SI_x$ for the stress index thus obtained corresponds to the stress index to be determined.

2. The method for detecting and reporting of a stress condition of a person according to claim 1, wherein normalization is each carried out by means of a normalization value

$$P_z = \quad P_{min} + a^*(P_{max} - P_{min})$$

$$HRV_z = HRV_{min} + b^* (HRV_{max} - HRV_{min})$$

and the calculation of the summands of the stress value SI is each carried out according to

$$SI_P = (P_{d1} - P_z)/(P_{max} - P_z) \text{ if } P_{d1} > P_z$$

$$SI_P = (P_{d1} - P_z)/(P_z - P_{min}) \text{ if } P_{d1} < P_z$$

$$SI_{HRV} = -(HRV_{d1} - HRV_z)/(HRV_{max} - HRV_z) \text{ if } HRV_{d1} > HRV_z$$

$$SI_{HRV} = -(HRV_{d1} - HRV_z)/(HRV_z - HRV_{min}) \text{ if } HRV_{d1} < HRV_z$$

and

$$SI_P = (P_{dx} - P_z)/(P_{max} - P_z) \text{ if } P_{dx} > P_z$$

$$SI_P = (P_{dx} - P_z)/(P_z - P_{min}) \text{ if } P_{dx} < P_z$$

$$SI_{HRV} = -(HRV_{dx} - HRV_z)/(HRV_{max} - HRV_z) \text{ if } HRV_{dx} > HRV_z$$

$$SI_{HRV} = -(HRV_{dx} - HRV_z)/(HRV_z - HRV_{min}) \text{ if } HRV_{dx} < HRV_z.$$

3. The method according to claim 2, wherein a is selected as 0.25 and b as 0.5.

4. The method according to one of claims 1 to 3, wherein c and d are selected as 1.

5. The method according to one of claims 1 to 4, wherein the current stress index SI is selected after the time interval $T_x$ or after the predetermined number of pulse beats by means of a digital low-pass filter $SI = f^*SI_x + (1 - f)^*SI_{x-1}$ with f between 0.05 and 0.5, preferably equal to 0.1.

6. The method according to one of claims 1 to 5, wherein the time intervals or the times during which which a predetermined number of pulse beats are measured, overlap.

7. The method according to one of claims 1 to 5, wherein the time intervals or the times in which a predetermined number of pulse beats are measured, have a fixed or variable distance between each other.

8. A device for detecting and reporting of a stress condition of a person, comprising:

• an acquisition device for continuously acquiring data of the current pulse frequency and of the current heart rate variability, wherein said acquisition device is a pulse sensor or an electrocardiographic sensor,
• a processing device for continuously processing the data of the current pulse frequency and of the current heart rate variability, and
• a comparator device for determining a stress index and for comparing the same with an alert criterion

**characterized in that**

the processing device is configured in such manner that within a first time interval $T_1$ or across a predetermined number of pulse beats a first value $SI_1$ for the stress index is determined by adding a value $SI_P$ for the stress index, which is obtained from a normalized average value $P_{d1}$ of the pulse frequency in the mentioned first time interval $T_1$ or across the predetermined number of pulse beats, plus a value $SI_{HRV}$, which is obtained from a normalized average value $HRV_{d1}$ of the heart rate variability HRV within the mentioned first time interval $T_1$ or across the predetermined number of pulse beats:

$$SI_1 = c^* \, SI_P + d^* \, SI_{HRV}$$

wherein c and d are weighting factors of the stress index partial value which is determined from the pulse frequency and of the stress index partial value which is determined from the heart rate variability, respectively,
wherein normalization is carried out by means of tabulated values $P_{max}$, $P_{min}$, $HRV_{max}$ and $HRV_{min}$ obtained from age dependent minimum and maximum pulse frequency values and HRV values, and, furthermore, the maximum and minimum values of the measured pulse frequency values and HRV values within the time interval $T_1$ or across the predetermined number of pulse beats are determined,
wherein $T_1$ lies between 100 s and 1000 s, and is preferably 300 s, or the predetermined number of pulse beats lies between 50 and 500, and is preferably 100,
in at least one further time interval $T_x (x = 2...n)$ or across a further predetermined number of pulse beats a further value $SI_x$ for the stress index is determined by adding a value $SI_P$ for the stress index, which is obtained from a normalized average value $P_{d1}$ of the pulse frequency in the mentioned time interval $T_x$ or across the predetermined number of pulse beats, plus a value $SI_{HRV}$, which is obtained from a normalized average value $HRV_{d1}$ of the heart rate variability HRV within the mentioned time interval $T_x$ or across the predetermined number of pulse beats:

$$SI_x = c^* \, SI_P + d^* \, SI_{HRV}$$

preferably having the same length as $T_1$ or having the same number of pulse beats, wherein c and d are again weighting factors of the stress index partial value which is determined from the pulse frequency and of the stress index partial value which is determined from the heart rate variability, respectively,
wherein normalization is carried out by means of values $P_{max}$, $P_{min}$, $HRV_{max}$ and $HRV_{min}$, wherein $P_{max}$ and $HRV_{max}$ are selected from the larger value of $P_{max}$ and $HRV_{max}$ determined in the previous time interval $T_{x-1}$ or across the predetermined number of pulse beats and the values of $P_{max}$ and $HRV_{max}$ used in the previous time interval $T_{x-1}$ or across the predetermined number of pulse beats, and wherein $P_{min}$ and $HRV_{min}$ are selected from the smaller value of $P_{min}$ and $HRV_{min}$ determined in the previous time interval $T_{x-1}$ or across the predetermined number of pulse beats and the values of $P_{min}$ and $HRV_{min}$ used in the previous time interval $T_{x-1}$ or across the predetermined number of pulse beats, wherein the further value $SI_x$ for the stress index thus obtained corresponds to the stress index to be determined.

9. The device according to claim 8, wherein the device is configured in such manner that the method can be carried out according to claim 2.

10. The device according to claim 8 or 9, wherein a is selected as 0.25 and b as 0.5 and/or wherein c and d are selected as 1, and/or wherein the current stress index SI is selected after the time interval $T_x$ or after the predetermined number of pulse beats by means of a digital low-pass filter $SI = f^*SI_x + (1-f)^*SI_{x-1}$
with f between 0.05 and 0.5, preferably 0.1.

**Revendications**

1. Procédé permettant d'identifier et de signaler un état de tension d'une personne ce procédé comprenant les étapes suivantes consistant à :

- détecter en continu des données de la fréquence du pouls P instantanée et de la variabilité du rythme cardiaque HRV instantanée au moyen d'un détecteur de mesure du pouls ou d'un détecteur électrocardiographique,
- traiter en continu les données de la fréquence du pouls P instantanée et de la variabilité du rythme cardiaque HRV instantanée,
- déterminer un indice de stress et le comparer à un critère d'alarme,

**caractérisé en ce que**

la détermination de l'état de tension comprend les étapes suivantes : pendant un premier intervalle de temps $T_1$ ou pour un nombre prédéfini de palpitations, une première valeur $SI_1$ de l'indice de stress est déterminée par addition d'une valeur $SI_P$ de l'indice de stress obtenue à partir d'une valeur moyenne, normalisée $P_{d1}$ du pouls, pendant le premier intervalle de temps $T_1$ ou pour un nombre prédéfini de palpitations, et d'une valeur $SI_{HRV}$ obtenue à partir d'une valeur moyenne normalisée $HRV_{d1}$ de la variabilité du rythme cardiaque HRV pendant le premier intervalle de temps $T_1$ ou pour le nombre prédéfini de palpitations selon la formule :

$$SI1 = c* \ SI_P + d* \ SI_{HRV}$$

dans laquelle c et d représentent des facteurs de pondération de la valeur partielle de l'indice de stress évaluée à partir du rythme cardiaque ou de la valeur partielle de l'indice de stress évaluée à partir de la variabilité du rythme cardiaque, la normalisation étant effectuée au moyen de valeurs tabulaires $P_{max}$, $P_{min}$, $HRV_{max}$ et $HRV_{min}$, déterminées au moyen de valeurs du pouls et de valeurs HRV minimum et maximum dépendant de l'âge, ainsi que des valeurs maximum et minimum de la valeur du pouls et de la valeur HRV mesurées pendant l'intervalle de temps $T_1$ ou pour le nombre prédéfini de palpitations,

$T_1$ étant compris entre 100s et 1000s, de préférence égal à 300s, et le nombre prédéfini de palpitations étant situé entre 50 et 500 et étant de préférence égal à 100,

pendant au moins un autre intervalle de temps $T_x$ (x=2...n) ou pour un autre nombre prédéfini de palpitations, une autre valeur $SI_x$ de l'indice de stress est déterminée par addition d'une valeur $SI_p$ de l'indice de stress obtenue à partir d'une valeur moyenne normalisée $P_{d1}$ du pouls pendant l'intervalle de temps $T_x$ ou pour le nombre prédéfini de palpitations, et d'une valeur $SI_{HRV}$ obtenue à partir d'une valeur moyenne normalisée $HRV_{d1}$ de la variabilité du rythme cardiaque HRV pendant l'intervalle de temps $T_x$ ou pour le nombre prédéfini de palpitations selon la formule :

$$SIx = c* \ SI_P + d* \ SI_{HRV}$$

de préférence avec un intervalle de temps de même longueur que $T_1$ ou le même nombre de palpitations, c et d représentant également des facteurs de pondération de la valeur partielle de l'indice de stress évaluée à partir du rythme cardiaque ou de la valeur partielle de l'indice de stress évaluée à partir de la variabilité du rythme cardiaque, la normalisation étant effectuée au moyen de valeurs $P_{max}$, $P_{min}$, $HRV_{max}$ et $HRV_{min}$, $P_{max}$ ou $HRV_{max}$ étant choisi parmi la plus grande valeur de Pmax ou de $HRV_{max}$ déterminée pendant l'intervalle de temps $T_{x-1}$, précédent ou pour le nombre prédéfini précédent de palpitations, et la valeur de $P_{max}$ ou de $HRV_{max}$ utilisée pendant l'intervalle de temps précédent $T_{x-1}$ ou pour le nombre prédéfini de palpitations, et $P_{min}$, ou $HRV_{min}$ étant choisi parmi la plus petite valeur de $P_{min}$ ou de $HRV_{min}$ déterminée pendant l'intervalle précédent $T_{x-1}$ ou pour le nombre prédéfini précédent de palpitations et la valeur de $P_{min}$ ou de $HRV_{min}$ utilisée pendant l'intervalle de temps prédéfini $T_{x-1}$ ou pour le nombre prédéfini précédent de palpitations, l'autre valeur ainsi obtenue $SI_x$ de l'indice de stress correspondant à l'indice de stress à déterminer.

**2.** Procédé permettant d'identifier et de signaler un état de tension d'une personne conforme à la revendication 1, selon lequel la normalisation est respectivement effectuée au moyen d'une valeur de normalisation :

$$P_z = P_{min} + a*(P_{max} - P_{min})$$

$$HRV_z = HRV_{min} + b* (HRV_{max} - HRV_{min})$$

et le calcul des additions pour la valeur de stress SI est respectivement effectué conformément aux formules

$$SI_p = (P_{d1} - P_z)/(P_{max} - P_z) \text{ si } P_{d1} > P_z$$

$$SI_p = (P_{d1} - P_z)/(P_z - P_{min}) \text{ si } P_{d1} < P_z$$

$$SI_{HRV} = -(HRV_{d1} - HRV_z)/(HRV_{max} - HRV_z) \text{ si } HRV_{d1} > HRV_z$$

$$SI_{HRV} = -(HRV_{d1} - HRV_z)/(HRV_z - HRV_{min}) \text{ si } HRV_{d1} < HRV_z$$

ou

$$SI_p = (P_{dx} - P_z)/(P_{max} - P_z) \text{ si } P_{dx} > P_z$$

$$SI_p = (P_{dx} - P_z)/(P_z - P_{min}) \text{ si } P_{dx} < P_z$$

$$SI_{HRV} = -(HRV_{dx} - HRV_z)/(HRV_{max} - HRV_z) \text{ si } HRV_{dx} > HRV_z$$

$$SI_{HRV} = -(HRV_{dx} - HRV_z)/(HRV_z - HRV_{min}) \text{ si } HRV_{dx} < HRV_z$$

3. Procédé conforme à la revendication 2,
   selon lequel a est égal à 0,25 et b est égal à 0,5.

4. Procédé conforme à l'une des revendications 1 à 3,
   selon lequel c et d sont égaux à 1.

5. Procédé conforme à l'une des revendications 1 à 4,
   selon lequel l'indice de stress actuel SI après l'intervalle de temps $T_x$ ou après le nombre prédéfini de palpitations est choisi au moyen d'un filtre passe bas numérique selon la formule $SI = f^*SI_x + (1-f)^*SI_{x-1}$ f étant compris entre 0,05 et 0,5 et de préférence égal à 0,1.

6. Procédé conforme à l'une des revendications 1 à 5,
   selon lequel les intervalles de temps ou les instants pour lesquels un nombre prédéfini de palpitations est mesuré se chevauchent.

7. Procédé conforme à l'une des revendications 1 à 5,
   selon lequel les intervalles de temps ou les instants pour lesquels un nombre prédéfini de palpitations est mesuré sont situés à une distance fixe ou variable.

8. Dispositif permettant d'identifier et de signaler un état de tension d'une personne comprenant :

   - un dispositif de détection permettant de détecter en continu les données de la fréquence du pouls instantanée et de la variabilité du rythme cardiaque instantanée, ce dispositif de détection étant un détecteur de mesure du pouls ou un détecteur électrocardiographique,
   - un dispositif de traitement permettant un traitement continu des données de la fréquence du pouls instantanée et de la variabilité du rythme cardiaque instantanée, et
   - un dispositif de comparaison permettant de déterminer un indice de stress et de le comparer à un critère d'alarme,

   **caractérisé en ce que**
   le dispositif de traitement est réalisé pour permettre de déterminer, pendant un premier intervalle de temps $T_1$ ou pour un nombre prédéfini de palpitations une première valeur $SI_1$ de l'indice de stress, par addition d'une valeur

$SI_P$ de l'indice de stress obtenue à partir d'une valeur moyenne normalisée $P_{d1}$ du pouls pendant le premier intervalle de temps $T_1$ ou pour le nombre prédéfini de palpitations, et d'une valeur $SI_{HRV}$ obtenue à partir d'une valeur moyenne normalisée $HRV_{d1}$ de la variabilité du rythme cardiaque HRV pendant le premier intervalle de temps $T_1$ ou pour le nombre prédéfini de palpitations, selon la formule :

$$SI_1 = c* \ SI_P + d* \ SI_{HRV}$$

dans laquelle c et d sont des facteurs de pondération de la valeur partielle de l'indice de stress évaluée à partir du rythme cardiaque ou de la valeur partielle de l'indice de stress évaluée à partir de la variabilité du rythme cardiaque, la normalisation étant effectuée au moyen de valeurs tabulaires $P_{max}$, $P_{min}$, $HRV_{max}$ et $HRV_{min}$ déterminées au moyen de valeurs du pouls et de valeurs HRV minimum et maximum dépendant de l'âge ainsi que des valeurs maximum et minimum de la valeur du pouls et de la valeur HRV mesurées pendant l'intervalle de temps $T_1$ ou pour le nombre prédéfini de palpitations,

$T_1$ étant compris entre 100s et 1000s, et étant de préférence égal 300s, ou le nombre prédéfini de palpitations étant compris entre 50 et 500 et étant de préférence égal à 100, et

de déterminer pendant au moins un autre intervalle de temps $T_x(x=2...n)$ ou pour un autre nombre prédéfini de palpitations, une autre valeur $SI_x$ de l'indice de stress par addition d'une valeur $SI_p$ de l'indice de stress obtenue à partir d'une valeur moyenne $P_{d1}$ normalisée du pouls pendant l'intervalle de temps $T_x$ ou pour le nombre prédéfini de palpitations, et d'une valeur $SI_{HRV}$ obtenue à partir d'une valeur moyenne $HRV_{d1}$ normalisée de la variabilité du rythme cardiaque HRV pendant l'intervalle de temps $T_x$ ou pour le nombre prédéfini de palpitations, selon la formule :

$$SI_x = c* \ SI_p + d* SI_{HRV}$$

de préférence avec un intervalle de temps de même longueur que $T_1$ ou le même nombre de palpitations, c et d étant également des facteurs de pondération de la valeur partielle de l'indice de stress évaluée à partir du pouls ou de la valeur partielle de l'indice de stress évaluée à partir de la variabilité du rythme cardiaque, la normalisation étant effectuée au moyen de valeurs $P_{max}$, $P_{min}$, $HRV_{max}$ et $HRV_{min}$, $P_{max}$ ou $HRV_{max}$ étant choisies parmi la plus grande valeur de Pmax ou HRVmax déterminée pendant l'intervalle de temps précédent $T_{x-1}$ ou pour le nombre prédéfini précédent de palpitations, et la valeur de $P_{max}$ ou de $HRV_{max}$ utilisée pendant l'intervalle de temps $T_{x-1}$ précédent ou pour le nombre prédéfini précédent de palpitations, et $P_{min}$ ou $HRV_{min}$ étant choisis parmi la plus petite valeur de $P_{min}$ ou de $HRV_{min}$ déterminée pendant l'intervalle de temps précédent $T_{x-1}$ ou pour le nombre prédéfini précédent de palpitations, et la valeur de $P_{min}$, ou de $HRV_{min}$ utilisée pendant l'intervalle de temps précédent $T_{x-1}$ ou pour le nombre prédéfini précédent de palpitations, l'autre valeur $SI_x$ ainsi obtenue de l'indice de stress correspondant à l'indice de stress à déterminer.

9. Dispositif conforme à la revendication 8,
   réalisé pour permettre la mise en œuvre du procédé conforme à la revendications 2.

10. Dispositif conforme à la revendication 8 ou 9,
    dans lequel a est égal à 0, 25 et b est égal à 0,5 et/ou c et d sont égaux à 1 et/ou l'indice de stress SI actuel après l'intervalle de temps $T_x$ ou après le nombre prédéfini de palpitations est choisi au moyen d'un filtre passe bas numérique selon la formule $SI-f*SI_x+(1-f)*SI_{x-1}$,
    étant f compris entre 0,05 et 0,5, et de préférence égal à 0,1.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10319361 A1
- DE 10006154 A1
- DE 102006039957 A1
- DE 102008030956 A1
- EP 1156851 B1
- EP 2316333 A1

**In der Beschreibung aufgeführte Nicht-Patentliteratur**

- **ANGELINK et al.** *Innovationstagung FH Rapperswil,* 04. Mai 2001